(19) Europäisches Patentamt
European Patent Office
Office européen des brevets

(11) EP 1 404 280 B1

(12) **FASCICULE DE BREVET EUROPEEN**

(45) Date de publication et mention de la délivrance du brevet: **28.12.2005 Bulletin 2005/52**

(21) Numéro de dépôt: **02762489.9**

(22) Date de dépôt: **13.06.2002**

(51) Int Cl.$^7$: **A61K 6/10**, C08L 83/04

(86) Numéro de dépôt international: **PCT/FR2002/002020**

(87) Numéro de publication internationale: **WO 2002/102326 (27.12.2002 Gazette 2002/52)**

(54) **MATERIAU ELASTOMERE SILICONE HYDROPHILE UTILISABLE NOTAMMENT POUR LA PRISE D'EMPREINTES DENTAIRES**

HYDROPHILES ELASTOMERES SILIKON-MATERIAL, INSBESONDERE ZUR ANFERTIGUNG VON DENTALABDRÜCKEN

HYDROPHILIC SILICONE ELASTOMERIC MATERIAL USED IN PARTICULAR FOR TAKING DENTAL IMPRINTS

(84) Etats contractants désignés: **AT BE CH CY DE DK ES FI FR GB GR IE IT LI LU MC NL PT SE TR**

(30) Priorité: **14.06.2001 FR 0107797**

(43) Date de publication de la demande: **07.04.2004 Bulletin 2004/15**

(73) Titulaire: **RHODIA CHIMIE**
**92512 Boulogne Billancourt Cedex (FR)**

(72) Inventeurs:
- **DEL TORTO, Marco**
**20151 Milano (IT)**
- **LEONARD, Yoan**
**42897 Remscheid (DE)**
- **PUSINERI, Christian**
**69360 Serezin du Rhône (FR)**

(74) Mandataire: **Trolliet, Maurice**
**RHODIA SERVICES**
**Direction de la Propriété Industrielle**
**CRIT-Carrières - BP 62**
**69192 Saint-Fons Cédex (FR)**

(56) Documents cités:
**EP-A- 0 480 238**
**FR-A- 2 791 996**
**GB-A- 2 314 849**
**GB-A- 2 337 524**
**US-A- 4 778 832**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 404 280 B1

Printed by Jouve, 75001 PARIS (FR)

## Description

Le domaine de la présente invention est celui des matériaux silicones comprenant une composition polyorganosiloxane (en abréviation POS) réticulable ou durcissable en élastomère silicone par des réactions de polyaddition ainsi qu'un agent mouillant permettant de conférer audit matériau un caractère hydrophile. Les applications visées par de tels systèmes sont, notamment, la prise d'empreintes et, plus particulièrement, la prise d'empreintes dentaires dans le cadre de la réalisation de prothèses. Par l'expression "prise d'empreintes", on entend définir dans le présent mémoire : non seulement les opérations de prise d'empreintes de n'importe quel objet et de n'importe quelle forme pour réaliser un modèle en particulier en plâtre ; mais encore les opérations de reproductions ou de duplications de modèles en particulier en plâtre. Par l'expression "prise d'empreintes dentaires", on entend définir dans le présent mémoire : non seulement les opérations où l'on procède à la prise d'empreintes dentaires en bouche pour obtenir des reproductions exactes de mâchoires ou de parties de mâchoires portant ou non, et en totalité ou en partie, des dents et pour former des modèles en plâtre ; mais encore les opérations de duplications où l'on procède à la reproduction de modèles de mâchoires ou de parties de mâchoires en plâtre dans un laboratoire de prothèse dentaire. Les applications visées englobent encore la fabrication de pièces moulées, autres que des masses de duplication dans les applications dentaires, capables de développer en surface un caractère hydrophile et/ou antistatique marqué.

La présente invention a également pour objet un procédé de préparation du matériau élastomère silicone hydrophile. L'invention vise encore l'utilisation dudit matériau pour la prise d'empreintes, par exemple dentaires et pour la fabrication de pièces moulées, autres que des masses de duplication dans les applications dentaires, capables de développer en surface un caractère hydrophile et/ou antistatique marqué.

L'utilisation des matériaux silicones est largement répandue dans ces domaines. Ceci est dû en partie au fait que les matériaux silicones offrent, d'une part une grande diversité de caractéristiques chimiques, mécaniques et physiques, et, d'autre part, un caractère non toxique, non irritant et non allergisant. En outre, les matériaux silicones constituent de piètres substrats de culture pour les micro-organismes, ce qui leur confère des aptitudes remarquables au regard de l'hygiène.

Les compositions POS auxquelles on s'intéresse dans le cadre de la présente invention comprennent au minimum :

- un POS (1) porteur de fonctions Si-alcényles aptes à réagir par des réactions d'addition avec les fonctions réticulantes Si-H d'un POS (2),
- un POS (2) porteur de fonctions Si-H apte à réagir avec les fonctions Si-alcényles du POS (1),
- éventuellement un POS (3) non réactif, différent des POS (1) et (2), utilisable comme diluant,
- un catalyseur des réactions de polyaddition, et
- une charge minérale renforçante particulaire et éventuellement une charge semi-renforçante ou de bourrage.

On sait que de pareilles compositions POS, pouvant avantageusement se présenter sous forme de deux composants, sont réticulables ou durcissables à température ambiante et sont particulièrement intéressantes dans le domaine des prises d'empreintes, en particulier des prises d'empreintes dentaires car ces compositions sont douées de propriétés de fluidité et de filmogénéité avant réticulation, ce qui rend possible la prise d'une empreinte de n'importe quelle forme avec une excellente reproduction des détails. Par ailleurs, ces compositions peuvent réticuler par des réactions de polyaddition en quelques minutes à température ambiante ; de plus, elles sont non toxiques et satisfont aux réglementations européennes en matière pharmaceutique. La réticulation qui entraîne le durcissement de la composition silicone permet de constituer des moules en élastomères offrant des propriétés mécaniques, une stabilité dimensionnelle et une tenue thermique qui sont conformes aux spécifications souhaitées.

Toutefois, les compositions pour empreintes à base de silicone réticulant par des réactions de polyaddition sont intrinsèquement hydrophobes. Ainsi donc, lorsqu'on applique la masse de moulage mélangée à la surface humide des dents et des gencives, il peut y avoir un défaut de coulée ou bien une pénétration insuffisante dans les creux des gencives en raison de la présence de résidus de liquides ; après réticulation, la reproduction est donc défectueuse. D'autre part, lorsqu'au cours des opérations de duplication du positif de l'empreinte en plâtre on est amené à couler, dans un moule en silicone hydrophobe, du plâtre réfractaire à caractère hydrophile, il peut y avoir occlusion de petites bulles d'air en raison de l'incompatibilité entre les surfaces ; cette occlusion conduit à la réalisation d'une reproduction défectueuse.

On peut pratiquement éliminer ces inconvénients en conférant aux compositions POS intrinsèquement hydrophobes, un caractère hydrophile grâce à l'emploi de différents agents tensioactifs ; c'est ainsi que l'on propose d'utiliser : dans US-A-4.657.959 : un polyorganosiloxane à fonctions polyéthers ; dans US-A-4.691.039 et US-A-4.752.633 : un silane éthoxylé ; dans US-A-5.064.891 : un polyorganosiloxane à fonctions polyols ; dans EP-A-0.480.238: un alcool gras poly(oxyalkylé) ; dans FR-A-2.600.886 : une protéine soluble dans l'eau associée éventuellement à un agent tensioactif non ionique.

Le caractère hydrophile est quantifié grâce à la mesure de l'angle de contact de gouttes d'eau qui sont déposées à la surface de films réticulés, de 2 mm d'épaisseur, réalisés à partir d'une composition POS de polyaddition. La mesure de l'angle est effectuée 3 minutes après avoir déposé les gouttes d'eau. Dans la figure 1 ci-jointe en annexe, le repère 1 représente la surface du film silicone réticulé, le repère 2 représente la goutte d'eau déposée sur ladite surface et le symbole θ représente l'angle de contact de la goutte avec la surface de dépôt, qui est mesuré.

En absence d'agent tensioactif, les angles de contact sont généralement de l'ordre de 100 à 105°C. Les agents tensioactifs, qui sont incorporés dans la composition POS de polyaddition, confèrent aux dites compositions et aux élastomères silicones réticulés qui en découlent, un caractère hydrophile qui se traduit par un abaissement de la valeur des angles de contact de gouttes d'eau qui sont déposées sur la surface des élastomères réticulés. Pour pouvoir être considérée comme hydrophile, la composition POS de polyaddition doit présenter des angles de contact inférieurs à 65°.

Dans un tel contexte technique, l'un des objectifs essentiels de la présente invention est de proposer un matériau silicone, utilisable notamment pour la prise d'empreintes, par exemple dentaires, qui permette d'atteindre, après réticulation, des angles de contact :

- égaux ou inférieurs à 40°,
- avec la possibilité d'atteindre des valeurs égales ou inférieures à 30°, et mieux encore
- avec la possibilité d'atteindre au besoin des valeurs aussi basses que celles allant de 25 à 27°.

Pour atteindre cet objectif, parmi d'autres, les inventeurs ont eu le mérite de mettre à jour de manière tout à fait surprenante et inattendue, un agent mouillant, judicieusement sélectionné, consistant dans des agents tensioactifs non ioniques particuliers et dans des associations synergiques d'agents tensioactifs non ioniques particuliers.

Plus précisément, la présente invention, prise dans son premier objet, concerne un matériau silicone, utilisable notamment pour la prise d'empreintes, par exemple dentaires, qui comprend les constituants suivants :

I. une composition POS réticulable par des réactions de polyaddition comprenant :

(1) au moins un POS porteur de fonctions Si-alcényles aptes à réagir par des réactions d'addition avec les fonctions réticulantes Si-H d'un POS (2),
(2) au moins un POS porteur de fonctions Si-H aptes à réagir avec les fonctions Si-alcényles du POS (1),
(3) éventuellement au moins un POS non réactif, différent des POS(1) et (2), utilisable comme diluant,
(4) un catalyseur des réactions de polyaddition,
(5) une charge minérale renforçante particulaire ;

**II.** un agent mouillant consistant dans un ou plusieurs agents(s) tensioactifs permettant de conférer un caractère hydrophile marqué à la surface du matériau silicone, avant comme après réticulation ; ledit matériau silicone étant caractérisé en ce que l'agent mouillant **II** est choisi dans le groupe constitué par :

- (a) au moins un agent tensioactif non ionique consistant dans un ester obtenu par estérification d'un monoacide carboxylique saturé à chaîne linéaire ou ramifiée en $C_{10}$-$C_{14}$ par un poly(oxyalkylène)glycol contenant un nombre de motifs alkoxylés tel que la masse molaire Mw de l'ester se situe dans l'intervalle allant de 400 à 800 g/mole, le HLB dudit agent tensioactif ou du mélange d'agents tensioactifs allant de 6 à14;
- une association synergique (a) + (b) où (b) désigne au moins un agent tensioactif non ionique consistant dans un alcool aliphatique saturé à chaîne linéaire ou ramifiée en $C_5$-$C_{16}$, polyalkoxylé contenant un nombre de motifs alkoxylés tel que la masse molaire Mw de l'alcool polyalkoxylé se situe dans l'intervalle allant de 200 à 1400 g/mole, le HLB dudit agent tensioactif (b) ou du mélange d'agents tensioactifs (b) allant de 6 à 16 ;
- une association synergique (b1) + (b2) où : (b1) désigne au moins un agent tensioactif non ionique consistant dans un alcool aliphatique saturé à chaîne linéaire ou ramifiée en $C_{10}$-$C_{16}$, polyéthoxylé contenant de 6 à 10 motifs oxyéthylène (OE), le HLB dudit agent tensioactif (b1) ou du mélange d'agents tensioactifs (b1) allant de 12 à 16 ; (b2) désigne au moins un agent tensioactif non ionique consistant dans un alcool aliphatique saturé à chaîne linéaire ou ramifiée en $C_6$-$C_{13}$, polyalkoxylé contenant un nombre de motifs oxyéthylène (OE) et oxypropylène (OP) tel que la masse molaire Mw de l'alcool polyalkoxylé se situe dans l'intervalle allant de 500 à 1200 g/mole, le HLB dudit agent tensioactif (b2) ou du mélange d'agents tensioactifs (b2) allant de 6 à 16.

De manière générale, la quantité d'agent tensioactif ou celle d'un mélange d'agents tensioactifs, exprimée en % en poids par rapport à la masse totale de la composition POS I, est égale ou supérieure à 1 % et, de préférence, se situe dans l'intervalle allant de 1,2 à 4 %, et plus préférentiellement encore se situe dans l'intervalle allant de 1,2 à 3 %.

Une modalité de mise en oeuvre de la présente invention qui est préférée pour atteindre des angles de contact égaux ou inférieurs à 40° (modalité 1), est l'utilisation, dans les quantités données supra, d'un agent mouillant choisi dans le groupe constitué par:

- (a1) au moins un agent tensioactif non ionique consistant dans un ester obtenu par estérification d'un monoacide carboxylique saturé à chaîne linéaire ou ramifiée en $C_{10}$-$C_{14}$ par un poly(oxyalkylène)glycol contenant de 7 à 11 motifs OE et/ou OP, le HLB dudit agent tensioactif ou du mélange d'agents tensioactifs allant de 10 à 14 ;
- une association synergique (a1) + (b1);
- une association synergique (a1) + (b2)
- une association synergique (a1) + (b3) où (b3) désigne au moins un agent tensioactif non ionique consistant dans un alcool aliphatique saturé à chaîne linéaire ou ramifiée en $C_{10}$-$C_{14}$, polyéthoxylé contenant de 1 à 4 motifs OE, le HLB dudit agent tensioactif (b3) ou du mélange d'agents tensioactifs (b3) allant de 6 à 10 ; et
- une association synergique (b1) + (b2).

Comme exemples spécifiques d'agents tensioactifs, on peut citer notamment les composés suivants :

(a) - (a1) :

. ester obtenu par estérification d'un acide gras en $C_{13}$ (acide laurique) par un poly(oxyéthylène)glycol contenant environ 9 motifs OE, ayant un HLB de 13,1, commercialisé sous la dénomination LINCOL PE 400 ML (en abréviation TA1) ;

(b) - (b1) :

. alcool aliphatique saturé en $C_{13}$ polyéthoxylé contenant environ 8 motifs OE, ayant un HLB de 12,8, commercialisé sous la dénomination RHODASURF ROX (en abréviation TA2) ;

(b) - (b2) :

. alcool aliphatique saturé en $C_8$ polyalkoxylé contenant un nombre de motifs OE et OP tel que la masse moléculaire Mw de l'alcool polyalkoxylé soit égale à environ 1000 g/mole, commercialisé sous la dénomination TEGOPREN LP 111 (en abréviation TA3) ;

. alcool aliphatique saturé en $C_{10}$ - $C_{12}$ polyalkoxylé contenant environ 4 motifs OE et 3 motifs OP, ayant un HLB de 7, commercialisé sous la dénomination ANTAROX FM 33 (en abréviation TA4) ;

(b) - (b3) :

. alcool aliphatique saturé en $C_{12}$ polyéthoxylé contenant environ 2 motifs OE, ayant un HLB de 8,1, commercialisé sous la dénomination RHODASURF OT/2 (en abréviation TA5).

Une modalité de mise en oeuvre de la présente invention qui est spécialement adaptée pour atteindre des angles de contact égaux ou inférieurs à 30° (modalité 2), est l'utilisation d'un agent mouillant choisi dans le groupe formé par :

- une association synergique (a1) + (b1),
- une association synergique (a1) + (b2), et
- une association synergique (b1) + (b2),

où :

- la quantité du mélange d'agents tensioactifs se situe dans l'intervalle allant de 1,2 à 3%,
- le rapport pondéral (a1)/(b1) se situe dans l'intervalle allant de 0,5 à 1,5,
- le rapport pondéral (a1)/(b2) se situe dans l'intervalle allant de 0,5 à 7 avec les trois conditions selon lesquelles : (i) quand ledit rapport se situe dans l'intervalle allant de 0,5 à 1,2, alors la quantité du mélange d'agents tensioactifs est inférieure à 1,8 % ; (2i) quand ledit rapport se situe dans l'intervalle allant de 3 à 7, alors la quantité du mélange d'agents tensioactifs est égale ou supérieure à 1,8 % ; et (3i) quand ledit rapport se situe dans l'intervalle allant d'une valeur supérieure à 1,2 à une valeur inférieure à 3, alors la quantité du mélange d'agents tensioactifs est égale à une valeur quelconque prise dans l'intervalle donné supra allant de 1,2 à 3 % ;

- le rapport pondéral (b1)/(b2) se situe dans l'intervalle allant de 0,5 à 1,5.

Une modalité de mise en oeuvre de la présente invention qui est spécialement adaptée pour atteindre des valeurs d'angle de contact aussi basses que celles allant de 25 à 27° (modalité 3), est l'utilisation d'un agent mouillant consistant dans une association synergique (a1) + (b2), où :

- la quantité du mélange d'agents tensioactifs se situe dans l'intervalle allant de 1,2 à 3%,
- le rapport pondéral (a1)/(b2) se situe dans l'intervalle allant de 0,7 à 1,7, avec les trois conditions selon lesquelles : (i) quand ledit rapport se situe dans l'intervalle allant de 0,7 à 1, alors la quantité du mélange d'agents tensioactifs est inférieure à 1,8 % ; (2i) quand ledit rapport se situe dans l'intervalle allant de 1,5 à 1,7, alors la quantité du mélange d'agents tensioactifs est égale ou supérieure à 1,8 % ; et (3i) quand ledit rapport se situe dans l'intervalle allant d'une valeur supérieure à 1 à une valeur inférieure à 1,5, alors la quantité du mélange d'agents tensioactifs est égale à une valeur quelconque prise dans l'intervalle donné supra allant de 1,2 à 3 %.

Une autre modalité de mise en oeuvre de la présente invention qui est spécialement adaptée pour atteindre des valeurs d'angle de contact aussi basses que celles allant de 25 à 27° (modalité 4), est l'utilisation d'un agent mouillant consistant dans une association synergique (b1) + (b2), où :

- la quantité du mélange d'agents tensioactifs se situe dans l'intervalle allant de 1,2 à 3%,
- le rapport pondéral (b1)/(b2) se situe dans l'intervalle allant de 0,8 à 1,2.

La modalité de mise en oeuvre de la présente invention, adaptée pour atteindre des valeurs d'angle de contact aussi basses que celles allant de 25 à 27°, qui donne les résultats les plus performants est celle (modalité 5) faisant appel à l'utilisation de l'association synergique (a1) + (b2) défine supra dans la modalité 3.

Comme exemples spécifiques de pareille association selon la modalité 5, on peut citer notamment :

- l'association synergique (a1) + (b2), où :
  - (a1) est l'agent tensioactif TA1,
  - (b2) est l'agent tensioactif TA3,
  - la quantité du mélange des agents tensioactifs est égale à 2 %,
  - le rapport pondéral (a1)/(b2) est égal à 1,6 ;
- l'association synergique (a1) + (b2), où :
  - (a1) est l'agent tensioactif TA1,
  - (b2) est l'agent tensioactif TA3,
  - la quantité du mélange des agents tensioactifs est égale à 1,75 %,
  - le rapport pondéral (a1)/(b2) est égal à 0,75 ;
- l'association synergique (a1) + (b2), où :
  - (a1) est l'agent tensioactif TA1,
  - (b2) est l'agent tensioactif TA3,
  - la quantité du mélange des agents tensioactifs est égale à 1,75 %,
  - le rapport pondéral (a1)/(b2) est égal à 1,33 ;
- l'association synergique (a1) + (b2), où :
  - (a1) est l'agent tensioactif TA1,
  - (b2) est l'agent tensioactif TA3,
  - la quantité du mélange des agents tensioactifs est égale à 1,875 %,
  - le rapport pondéral (a1)/(b2) est égal à 1,15.

Il ressort de ce qui précède que la composition POS I comprend les consitutants suivants :

(1) au moins un POS porteur de fonctions Si-alcényles aptes à réagir par des réactions d'addition avec les fonctions réticulantes Si-H d'un POS (2),

(2) au moins un POS porteur de fonctions Si-H aptes à réagir avec les fonctions Si-alcényles du POS (1),
(3) éventuellement au moins un POS non réactif, différent des POS (1) et (2), utilisable comme diulant,
(4) un catalyseur des réactions de polyaddition,
(5) une charge minérale renforçante particulaire.

En ce qui concerne les POS (1), il s'agit de polyorganosiloxanes qui présentent, par molécule, au moins deux groupes alcényles en $C_2$-$C_6$ liés au silicium, ces groupes étant situés dans la chaîne et/ou en bout(s) de chaîne.
Plus précisément, il s'agit de POS comprenant :

(i) des motifs siloxyles de formule :

$$T_a\, Z_b\, SiO_{\frac{4-(a+b)}{2}} \qquad (1.1)$$

dans laquelle :

- T est un groupe alcényle en $C_2$-$C_6$, de préférence vinyle ou allyle,
- Z est un groupe hydrocarboné monovalent, exempt d'action défavorable sur l'activité du catalyseur et choisi, de préférence, parmi les groupes alkyles ayant de 1 à 8 atomes de carbone inclus, éventuellement substitués par au moins un atome d'halogène, avantageusement, parmi les groupes méthyle, éthyle, propyle et 3,3,3-trifluoropropyle et ainsi que parmi les groupes aryles et, avantageusement, parmi les radicaux xylyle, tolyle et phényle,
- a est 1 ou 2, b est 0, 1 ou 2 et a + b est compris entre 1 et 3, de préférence entre 2 et 3,

et (2i) éventuellement des autres motifs siloxyles de formule :

$$Z_c\, SiO_{\frac{4-c}{2}} \qquad (1.2)$$

dans laquelle Z a la même signification que ci-dessus et c a une valeur comprise entre 0 et 3, de préférence entre 2 et 3.

Il est avantageux que ce POS ait une viscosité comprise entre 200 et 20 000 mPa.s, et de préférence entre 500 et 5000.
Bien entendu, en cas de mélange de plusieurs huiles (1) de viscosité différentes, on prend en compte la viscosité du mélange.
Toutes les viscosités dont il est question ici correspondent à une grandeur de viscosité dynamique qui est mesurée, de manière connue en soi, à 25°C.
Le POS (1) peut être uniquement formé de motifs de formule (1.1) ou peut contenir, en outre, des motifs de formule (1.2). De même, il peut présenter une structure linéaire, ramifiée, cyclique ou en réseau.
Z est généralement choisi parmi les radicaux méthyle, éthyle et phényle, 60 % molaire (ou en nombre) au moins des radicaux Z étant des radicaux méthyle.
Des exemples de motifs siloxyles de formule (1.1) sont le motif vinyldiméthylsiloxyle, le motif vinylphénylméthylsiloxyle, le motif vinylméthylsiloxyle et le motif vinylsiloxyle.
Des exemples de motifs siloxyles de formule (1.2) sont les motifs $SiO_{4/2}$, diméthylsiloxyle, méthylphénylsiloxyle, diphénylsiloxyle, méthylsiloxyle et phénylsiloxyle.
Des exemples de POS (1) sont des composés linéaires et cycliques comme : les diméthylpolysiloxanes à extrémités diméthylvinylsilyles, les copolymères (méthylvinyl) (diméthyl)polysiloxanes à extrémités triméthylsilyles, les copolymères (méthylvinyl) (diméthyl)polysiloxanes à extrémités diméthylvinylsilyles ; les méthylvinylpolysiloxanes cycliques.
En ce qui concerne les POS (2), il s'agit de polyorganosiloxanes qui présentent, par molécule, au moins deux atomes d'hydrogène liés au silicium, ces groupes Si-H étant situés dans la chaîne et/ou en bout de chaîne.
L'homme du métier sait bien que quand le POS (1) a 2 groupes alcényles par molécule, le POS (2) doit avoir de préférence au moins 3 atomes d'hydrogène par molécule. Inversement, lorsque le POS (2) a 2 atomes d'hyrogène par molécule, le POS (1) a de préférence au moins 3 groupes alcényles par molécule.
Le POS (2) est plus précisément un polyorganosiloxane comprenant :

(i) des motifs siloxyles de formule :

$$H_d\ L_e\ SiO_{\frac{4-(d+e)}{2}} \qquad (2.1)$$

dans laquelle :

- L est un groupe hydrocarboné monovalent, exempt d'action défavorable sur l'activité du catalyseur et choisi, de préférence, parmi les groupes alkyles ayant de 1 à 8 atomes de carbone inclus, éventuellement substitués par au moins un atome d'halogène, avantageusement, parmi les groupes méthyle, éthyle, propyle et 3,3,3-trifluoropropyle et ainsi que parmi les groupes aryles et, avantageusement, parmi les radicaux xylyle, tolyle et phényle,
- d est 1 ou 2, e est 0, 1 ou 2, d + e a une valeur comprise entre 1 et 3, de préférence entre 2 et 3,

et (2i) éventuellement des autres motifs siloxyles de formule moyenne :

$$L_g\ SiO_{\frac{4-g}{2}} \qquad (2.2)$$

dans laquelle L a la même signification que ci-dessus et g a une valeur comprise entre 0 et 3, de préférence entre 2 et 3.

La viscosité dynamique de ce polyorganosiloxane (2) est au moins égale à 10 mPa.s et, de préférence, elle est comprise entre 20 et 1000 mPa.s.

Le POS (2) peut être uniquement formé de motifs de formule (2.1) ou comporter en plus des motifs de formule (2.2).

Le polyorganosiloxane (2) peut présenter une structure linéaire, ramifiée, cyclique ou en réseau.

Le groupe L a la même signification que le groupe Z ci-dessus.

Des exemples de motifs de formule (2.1) sont :
$H(CH_3)_2SiO_{1/2}$, $HCH_3SiO_{2/2}$, $H(C_6H_5)SiO_{2/2}$

Les exemples de motifs de formule (2.2) sont les mêmes que ceux donnés plus haut pour les motifs de formule (1.2).

Des exemples de POS (2) sont des composés linéaires et cycliques comme :

- les diméthylpolysiloxanes à extrémités hydrogénodiméthylsilyle,
- les copolymères (diméthyl)(hydrogénométhyl)polysiloxanes à extrémités triméthylsilyles,
- les copolymères (diméthyl)(hydrogénométhyl)polysiloxanes à extrémités hydrogénodiméthylsilyles,
- les hydrogénométhylpolysiloxanes à extrémités triméthylsilyles,
- les hydrogénométhylpolysiloxanes cycliques.

Le rapport du nombre d'atomes d'hydrogène liés au silicium dans le POS (2) sur le nombre total de groupes à insaturation alcényle du POS (1) est compris entre 0,4 et 10, de préférence entre 1 et 5.

En ce qui concerne les POS (3) non réactifs, utilisables comme diluants, il peut s'agir avantageusement d'un polydiorganosiloxane tel qu'un polydialkylorganosiloxane à extrémités trialkylsilyles ; on préfère les polydiméthylsiloxanes à extrémités triméthylsilyles. La viscosité dynamique à 25°C des POS (3) est comprise entre 10 et 5000 mPa.s et; de préférence, entre 20 et 1000 mPa.s. Ces POS (3), quand on en utilise, sont présents à raison de 10 à 120 parties en poids et, de préférence, de 20 à 100 parties en poids pour 100 parties des POS (1) et (2).

En ce qui concerne les catalyseurs (4) des réactions de polyaddition, ils sont bien connus de l'homme de métier.

On utilise, de préférence, les composés du platine et du rhodium. On peut, en particulier, utiliser les complexes du platine et d'un produit organique décrit dans les brevets US-A-3 159 601, US-A-3159 602, US-A-3 220 972 et les brevets européens EP-A-0 057 459, EP-A-0 188 978 et EP-A-0 190 530, les complexes du platine et d'organosiloxanes vinylés décrits dans les brevets US-A-3 419 593, US-A-3 715 334, US-A-3 377 432 et US-A-3 814 730. Le catalyseur plus spécialement préféré est à base de platine. Dans ce cas, la quantité pondérale de catalyseur (4), calculée en poids de platine-métal, est généralement comprise entre 2 et 400 ppm, de préférence entre 5 et 200 ppm basés sur le poids total des POS (1) et (2).

En ce qui concerne la charge minérale renforçante particulaire (5), la charge habituellement utilisée consiste

dans une charge siliceuse. A titre de charges siliceuses susceptibles d'être mises en oeuvre, conviennent toutes les silices précipitées ou pyrogénées (ou silices de combustion) connues de l'homme de l'art. Bien entendu, on peut utiliser aussi des coupages de différentes silices.

On préfère les silices de précipitation et/ou les silices de combustion ayant une surface spécifique BET supérieure à 40 $m^2/g$ et, plus précisément comprise entre 50 et 300 $m^2/g$. A titre plus préférentiel, on utilise les silices de combustion ayant les caractéristiques de surface spécifique mentionnées ci-avant. A titre encore plus préférentiel, on utilise les silices de combustion ayant une surface spécifique BET comprise entre 170 et 230 $m^2/g$. Généralement, cette charge renforçante présente une dimension moyenne des particules inférieures à 0,1 μm.

Ces silices peuvent être incorporées telles quelles ou après avoir été traitées par des composés orgnaosiliciques habituellement utilisés pour cet usage. Parmi ces composés figurent les méthylpolysiloxanes tels que l'hexaméthyldisiloxane, l'octaméthyldisiloxane, l'octaméthylcyclotétrasiloxane, des méthylpolysilazanes tels que l'hexaméthyldisilazane, l'hexaméthylcyclotrisilazane, des chlorosilanes tels que le diméthylchlorosilane, le triméthylchlorosilane, le méthylvinyldichlorosilane, le diméthylvinylchlorosilane, des alcoxysilanes tels que le diméthyldiméthoxysilane, le diméthylvinyléthoxysilane, le triméthylméthoxysilane.

Lors de ce traitement, les silices peuvent accroître leur poids de départ jusqu'à un taux de 20 % de préférence 18 % environ.

A noter que la charge minérale siliceuse particulaire peut avantageusement être mise en oeuvre sous forme de la suspension obtenue en traitant la charge par application de la méthode, conforme à l'enseignement des demandes de brevets WO-A-98/58997 et WO-A-00/00853, prévoyant un traitement en deux temps de la charge par un agent de compatibilisation (choisi par exemple : s'agissant du premier temps de traitement, parmi un silazane, un siloxane hydroxylé, une amine, un acide organique ; et s'agissant du second temps de traitement, parmi un silazane) en opérant en présence du constituant POS (1). Dans le cas où pareil traitement conduit à un pH basique, on peut ajouter dans la dispersion un neutralisant tel que par exemple un acide faible. Pareil traitement particulier de la charge est intéressant quand on a besoin de conserver une excellente fluidité pour le matériau silicone (à l'état non réticulé).

Ces charges sont présentes à raison de 2 à 30 %, de préférence de 3 à 20 % par rapport à la masse totale de la composition POS I.

Suivant une disposition avantageuse de la présente invention, la composition POS I du matériau silicone peut comprendre en outre un ou plusieurs constituants complémentaires choisis dans le groupe comprenant :

(6) au moins un inhibiteur des réactions de polyaddition,
(7) une charge semi-renforçante ou de bourrage,
(8) un ou plusieurs agent(s) de coloration, et/ou agent(s) édulcorant(s) et/ou arôme(s) et/ou composé(s) isotonique (s),
(9) un ou plusieurs biocide(s), et
(10) leurs mélanges.

Les inhibiteurs (6) sont des composés bien connus. On peut en particulier utiliser les amines organiques, les oximes organiques, des diesters de diacide carboxylique, les alcools acétyléniques, les cétones acétyléniques, les vinylméthylcyclopolysiloxanes (voir par exemple US-A-3.445.420 et US-A-3.989.667). Les alcools acétyléniques sont préférés et; dans ce contexte, l'éthynylcyclohexanol (ECH) est un inhibiteur particulièrement préféré. La concentration en inhibiteur(s), quand on en utilise, est au plus égale à 2000 ppm et, de préférence, est comprise entre 2 et 500 ppm par rapport à la masse totale des POS (1) et (2).

Pour ce qui concerne les charges (7), elles ont généralement un diamètre particulaire supérieur à 0,1 μm et elles sont choisies de préférence parmi le quartz broyé, les zircones, les argiles calcinées, les terres de diatomées, le carbonate de calcium, les silicates d'aluminium et/ou de sodium, des alumines, de l'oxyde de titane et des mélanges de ces espèces. Sur le plan pondéral, les charges (7), quand on en utilise, sont présentes dans le matériau silicone à raison de 5 à 60 % et, de préférence, de 30 à 50 % par rapport à la masse totale de la composition POS **I**.

En ce qui concerne le (ou les) agent(s) de coloration(s) (8), on peut utiliser des pigments colorés minéraux et/ou organiques.

En ce qui concerne l'agent biocide (9) qui peut être mis en oeuvre dans le matériau silicone selon l'invention, il est à noter qu'il est, de préférence, choisi dans le groupe de précurseur de chlore actif à base de composés N-chlorés comprenant :

- la chloramine B (sodium-N-chlorobenzène sulfonamide),
- la chloroamine T (sodium N-chloro-p-toluène sulfonamide),
- la dichloroamine T (N,N-dichloro-p-toluène sulfonamide),
- la N-trichlorométhylmercapto-4-cyclohexène-1,2-dicarboxylamide,
- l'halazone (acide benzoïque p-n-dichlorosulfonamide),

- la N-chlorosuccinimide,
- la trichloromélamine,
- la chloroazodine

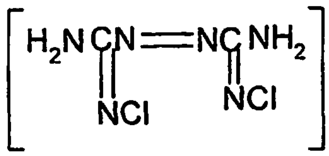

- les dérivés N-chloro des acides cyanuriques, de préférence l'acide trichloroisocyanurique et/ou le sodium dichloroisocyanurique dihydrate,
- les N-chlorohydantoïnes, de préférence la 1-bromo-3-chloro-5,5'-diméthylhydantoïne, ou la 1,3-dichloro-5,5'-diméthylhydantoïne,
- et leurs mélanges.

Ce groupe d'antiseptiques correspond sensiblement à la famille des N-chloramines qui comprend les dérivés des amines dans lesquelles une ou deux des valences de l'azote trivalent sont substituées par du chlore. En présence d'eau, les N-chloramines produisent de l'acide hypochloreux HClO ou des sels de cet acide tels que NaClO. HClO est NaClO sont des dérivés chlorés actifs, doués d'une grande capacité bactéricide, que l'on peut exploiter dans le cadre du matériau silicone selon l'invention (c'est en particulier le cas, lorsque ledit matériau est destiné à la prise d'empreintes dentaires en bouche).

Avantageusement, l'agent biocide (9) peut être associé à au moins un auxiliaire antiseptique différent des antiseptiques fonctionnant par libération de chlore et de préférence choisi dans le groupe des formulations comportant un ou plusieurs ammoniums quaternaires (par exemple, le chlorure de benzalkonium) et éventuellement au moins un activateur séquestrant, de préférence sélectionné parmi les complexants d'ions métalliques (par exemple, l'EDTA ou Acide Ethylène Diamine Tétracétique).

La concentration en agent(s) biocide(s), quand on en utilise, est au plus égale à 1 %, de préférence au plus égale à 0,8 %, et plus préférentiellement encore comprise entre 0,001 et 0,5 % en poids par rapport à la masse totale du matériau silicone [ensemble **I** + **II**].

La présente invention concerne également, dans un deuxième objet, un procédé de préparation du matériau silicone I + II tel que décrit ci-dessus. Ce procédé est caractérisé en ce qu'il consiste essentiellement à mélanger les ingrédients suivants :

(A) un ou plusieurs POS (1) tels que définis supra,
(B) un ou plusieurs POS (2) tels que définis supra,
(C) éventuellement un ou plusieurs POS (3) tels que définis supra,
(D) un catalyseur (4) des réactions de polyaddition,
(E) une charge minérale renforçante telle que définie supra,
(F) éventuellement un ou plusieurs inhibiteur(s) (6) tels que définis supra,
(G) éventuellement une charge semi-renforçante ou de bourrage (7) telle que définie supra,
(H) éventuellement un ou plusieurs agent(s) (8),
(I) éventuellement un ou plusieurs agent(s) biocide(s) (9), et
(J) un ou plusieurs agent(s) tensioactif(s) **II** tels que définis supra.

Ce mélange s'effectue de manière traditionnelle par les moyens techniques appropriés connus de l'homme de métier.

Selon une proposition avantageuse, il est préférable que la présence de l'ingrédient (G) soit rendue obligatoire.

Selon une variante intéressante de ce procédé :

- on produit le matériau silicone sous forme d'un système à deux composants A et B destinés à être mis en contact l'un avec l'autre pour produire un élastomère réticulé par réactions de polyaddition entre les POS (1) et (2), et
- on fait en sorte que l'un seulement des composants A et B comprenne le catalyseur (D) et éventuellement l'un ou l'autre des POS (1) et (2).

Selon une modalité préférée de la variante du procédé décrite ci-avant, on fait en sorte que l'agent tensioactif ou le mélange d'agents tensioactifs soit introduit dans le composant A ou B qui ne compend pas le catalyseur (D).

La présente invention a encore pour objet l'utilisation du matériau silicone **I** + **II**, tel que décrit ci-dessus, pour la prise d'empreintes, par exemple dentaires. Cette utilisation, dans une modalité de réalisation préférée, consiste à faire en sorte que la réticulation de l'élastomère silicone s'initie par mélange des composants A et B, à prendre l'empreinte et à laisser se poursuivre la réticulation jusqu'à ce que l'élastomère ait suffisamment réticulé et soit suffisamment dur.

Selon un autre mode d'utilisation, le matériau silicone **I** + **II** tel que décrit ci-dessus est destiné à la fabrication de pièces moulées, autres que des masses de duplication dans les applications dentaires, capables de développer en surface un caractère hydrophile et/ou antistatique marqué ; lesdites pièces moulées sont fabriquées selon par exemple des procédés de moulage par coulée ou des procédés de moulage par injection. A noter que, dans le cas des procédés de moulage par coulée où il est préférable que le matériau silicone puisse conserver une excellente fluidité à l'état non réticulé, il est avantageux alors de faire appel au traitement particulier de la charge siliceuse en deux temps par un agent de compatibilisation, dont on a parlé ci-avant lors de la définition de la charge minérale renforçante particulaire (5).

Comme exemples spécifiques de pièces moulées, autres que des masses de duplication dans les applications dentaires, on peut citer notamment des tampons tels que ceux utilisés dans les techniques de tampographie et des rouleaux de photocopieurs.

Dans le cadre de cet autre mode d'utilisation, le matériau silicone **I** + **II** tel que décrit ci-dessus est particulièrement destiné à la fabrication des tampons tels que ceux utilisés dans les techniques de tampographie, où il est intéressant de pouvoir disposer d'un matériau ayant de hautes propriétés mécaniques, dont on peut moduler l'énergie de surface par ajout d'agent(s) tensioactif(s) tout en conservant le niveau de fluidité qui est nécessaire à la fabrication des tampons par moulage, de préférence par coulée. Cette autre utilisation particulière, dans une modalité préférée de réalisation, consiste à faire en sorte que la réticulation de l'élastomère silicone s'initie par mélange des composants A et B, à conformer par moulage un objet ayant la forme du tampon souhaité et à laisser se poursuivre la réticulation jusqu'à ce que l'élastomère ait suffisamment réticulé et soit suffisamment dur.

Bien que la réticulation par des réactions de polyaddition entre les POS (1) et (2) puisse être initiée et développée déjà à une température voisine de la température ambiante (23°C), on peut également réaliser la réticulation par voie thermique (en chauffant par exemple à une température allant de 60°C à 110°C) et/ou par rayonnement électromagnétique (rayonnement d'électrons accélérés ou "electron beam") et/ou par rayonnement infrarouge.

L'invention sera mieux comprise à l'aide de l'exemple qui suit et qui décrit la préparation d'un matériau silicone selon l'invention, ainsi que son évaluation en termes de fluidité, d'hydrophilie et de propriétés mécaniques.

<u>EXEMPLES</u>

<u>Exemples 1 à 26 et essais témoins 1 à 5</u>

**1. Liste des matrières premières utilisées :**

1.1 Composant A du bicomposant :

- POS (1) : huile polydiméthylsiloxane bloquée à chacune des extrémités des chaînes par un motif $(CH_3)_2ViSiO_{1/2}$, de viscosité 600 mPa.s et contenant environ 0,014 fonction vinyle (Vi) dans 100 g d'huile ;
- catalyeur (4) : platine zéro complexé par du divinyltétraméthyldisiloxane : on engage une solution dans le divinyltétraméthyldisiloxane d'un complexe de platine à environ 11 % en poids de platine zéro ligandé (catalyseur dit de Karstedt) ;
- charge (5) : silice de combustion, commercialisée sous la dénomination AEROSIL R 972, traitée avec de l'octaméthylcyclotétrasiloxane ;
- charge (7) : quartz broyé de diamètre particulaire moyen de 10 µm, commercialisé sous la dénomination SICRON SA 600 ;
- base colorante blanche (8) : $TiO_2$ en dispersion (60 % en poids de $TiO_2$) dans une fraction d'huile POS (1).

1.2 Composant B du bicomposant :

- POS (1) : cf. composant A ;
- POS (1') : huile polydiméthylsiloxane bloquée à chacune des extrémités des chaînes par un motif $(CH_3)_2ViSiO_{1/2}$,

de viscosité 3500 mPa.s et contenant environ 0,0074 fonction vinyle (Vi) dans 100 g d'huile ;

- POS (2) : huile poly(diméthyl)(hydrogénométhyl)siloxane bloquée à chacune des extrémités des chaînes par un motif $(CH_3)_2HSiO_{1/2}$, ayant une viscosité de 30 mPa.s et contenant environ 0,25 fonction SiH dans 100 g d'huile;
- charge (5) : cf. composant A ;
- charge (7) : cf. composant B ;
- base colorante jaune (8) : mélange de 30% en poids de jaune de quinoléine dans 70% en poids d'huile POS (1') ;
- agent mouillant **II** : cf. légendes (1) à (6) jointes aux tableaux 1, 2 et 3 donnés ci-après.

**2. Constitution des composants A et B des bicomposants testés :**

| | Composant A | Composant B |
|---|---|---|
| Composition POS **I**: | | |
| - POS (1) | 49,5 (*) | 38,8 |
| - POS (1') | --- | 2,7 |
| - POS (2) | --- | 9,1 |
| - Platine zéro ligandé (4) | 0,015 | --- |
| - Divinyltétraméthyldisiloxane (4) | 0,035 | --- |
| - Charge (5) | 3,75 | 3,6 |
| - Charge (7) | 45,7 | 44,6 |
| - $TiO_2$ (8) | 1,0 | --- |
| - jaune de quinoléine (8) | --- | 1,2 |
| Agent(s) tensioactif(s) **II** | --- | cf. tableaux 1, 2 et 3 donnés ci-après (**) |

(*) : parties en poids ; la somme de constituants de la composition POS I est égale à 100 parties.

(**) : la quantité d'agent(s) tensioactif(s) introduite dans le composant B du bicomposant correspond aux valeurs indiquées dans les tableaux (ligne : % en poids dans A + B) multipliées par un facteur de 2.

**3. Préparation des compositions :**

3.1 Composant A :

Dans un mélangeur planétaire, on introduit à 23°C les constituants suivants : POS (1), charge (7), charge (5) et base colorante (8) ; l'ensemble est homogénéïsé par agitation à 400 tours/minute pendant 2 heures;

L'agitation est ensuite arrêtée et on ajoute alors le catalyseur de Karstedt (4) ; nouvelle homogénéïsation par agitation à 400 tours/minute pendant 10 minutes.

Puis, sans arrêter l'agitation, on procède au dégazage de la masse en opérant à 23°C, sous une pression réduite de $266.10^2$Pa pendant 10 minutes.

3.2 Composant B :

Dans le mélangeur précédent, on réalise les étapes suivantes :

Introduction des POS (1), charge (7), charge (5) et base colorante (8) à base de jaune de quinoléine et d'huile POS (1') et homogénéïsation à 23°C sous une agitation de 50 tours/minute pendant 2 heures.

Arrêt agitation et ajout du POS (2) et du (ou des) agent(-s) tensioactifs(s) ; nouvelle homogénéïsation par agitation à 50 tours/minute pendant 1 heure.

Enfin dégazage comme indiqué ci-avant pour le composant A, mais en agitant à 50 tours/minutes

3.3 Bicomposant A + B :

Le matériau silicone selon l'invention est obtenu par mélange, à température ambiante de 23°C, de 50 parties en poids du composant A avec 50 parties en poids du composant B. La réticulation de chaque bicomposant s'effectue à température ambiante de 23°C, après réalisation du mélange A + B.

**4. Résultats :**

Ils sont rassemblés dans les tableaux 1, 2 et 3 qui suivent :

TABLEAU 1

| | Témoin 1 | Témoin 2 | Ex.1 | Ex.2 | Ex.3 | Témoin 3 | Ex.4 | Ex.5 | Ex.6 | Témoin 4 | Ex.7 | Ex.8 | Témoin 5 |
|---|---|---|---|---|---|---|---|---|---|---|---|---|---|
| Agent(s) tensioactif(s) | | | | | | | | | | | | | |
| • nature : (1 à 6) | -- | TA6 | TA1 | TA1 | TA1 + TA2 | TA2 | TA1 + TA3 | TA1 + TA3 | TA1 + TA4 | TA4 | TA1 + TA5 | TA2 + TA3 | TA3 |
| • % en poids du (ou des) agent(s) tensioactif(s) dans A+B : (7) | -- | 2 | 2 | 3 | 2 | 2 | 2 | 2 | 2 | 2 | 2 | 2 | 2 |
| • rapport pondéral des agents tensioactifs : (8) | --- | --- | --- | --- | 1 | --- | 1 | 1,6 | 1 | --- | 1 | 1 | --- |
| Cinétique de réticulation | | | | | | | | | | | | | |
| • temps de travail : | 1'15" | 1'30" | 2'35" | 2'15" | 2'35" | 2'25" | 2'35" | 2'20" | 2'10" | 2'15" | 2'30" | 2'30" | 2'35" |
| • temps de prise : (9) | 2'15" | 2'22" | 4'20" | 3'10" | 3'35" | 3'55" | 4'20" | 4'10" | 3'55" | 3'55" | 4'20" | 4'25" | 4'35" |
| Dureté Shore A | | | | | | | | | | | | | |
| • 8 min : | 49 | 49 | 48 | 49 | 46 | 46 | 47 | 47 | 49 | 47 | 49 | 47 | 48 |
| • 24 heures : (10) | 50 | 51 | 49 | 50 | 48 | 49 | 49 | 49 | 51 | 50 | 49 | 49 | 49 |
| Angle de contact (11) | 105° | 61,4° | 38° | 36,2° | 29° | 41,7° | 32,5° | 25° | 33° | 45° | 30,5° | 27° | 48,1° |

TABLEAU 2

| | Ex.9 | Ex.10 | Ex.11 | Ex.12 | Ex.13 | Ex.14 | Ex.15 | Ex.16 | Ex.17 | Ex.18 |
|---|---|---|---|---|---|---|---|---|---|---|
| Agent(s) tensioactif(s) | | | | | | | | | | |
| • nature : (1 à 6) | TA1 + TA3 | TA1 + TA3 | TA1 + TA3 | TA1 + TA3 | TA1 + TA3 | TA1 + TA3 | TA1 + TA3 | TA1 + TA3 | TA1 + TA3 | TA1 + TA3 |
| • % en poids du (ou des) agent(s) tensioactif(s) dans A+B : (7) | 1,25 | 1,25 | 1,375 | 1,375 | 1,5 | 1,5 | 1,6 | 1,6 | 1,75 | 1,75 |
| • rapport pondéral des agents tensioactifs : (8) | 0,25 | 4 | 0,375 | 2,65 | 0,5 | 2 | 0,62 | 1,6 | 0,75 | 1,33 |
| Cinétique de réticulation | | | | | | | | | | |
| • temps de travail : | 2'20" | 2'20" | 2'25" | 2'20" | 2'25" | 2'20" | 2'35" | 2'20" | 2'30" | 2'25" |
| • temps de prise : (9) | 4' | 4' | 4'05" | 4' | 4' | 3'55" | 4'35" | 4' | 4'30" | 4'30" |
| Dureté Shore A | | | | | | | | | | |
| • 8 min : | 48 | 48 | 48 | 47 | 48 | 47 | 47 | 47 | 48 | 48 |
| • 24 heures : (10) | 49 | 49 | 49 | 49 | 50 | 49 | 48 | 49 | 49 | 49 |
| Angle de contact (11) | 34,5° | 33° | 30,5° | 34° | 30° | 30° | 28,5° | 29° | 25,5° | 25,5° |

TABLEAU 3

| | Ex.19 | Ex.20 | Ex.21 | Ex.22 | Ex.4 | Ex.5 | Ex.23 | Ex.24 | Ex.25 | Ex.26 |
|---|---|---|---|---|---|---|---|---|---|---|
| Agent(s) tensioactif(s) | | | | | | | | | | |
| • nature : (1 à 6) | TA1 + TA3 | TA1 + TA3 | TA1 + TA3 | TA1 + TA3 | TA1 + TA3 | TA1 + TA3 | TA1 + TA3 | TA1 + TA3 | TA1 + TA3 | TA1 + TA3 |
| • % en poids du (ou des) agent(s) tensioactif(s) dans A+B : (7) | 1,875 | 1,875 | 2 | 2 | 2 | 2 | 2 | 2 | 2 | 2 |
| • rapport pondéral des agents tensioactifs : (8) | 0,875 | 1,15 | 0,14 | 0,6 | 1 | 1,6 | 2 | 3 | 4 | 7 |
| Cinétique de réticulation | | | | | | | | | | |
| • temps de travail : | 2'35" | 2'30" | 2'44" | 2'35" | 2'35" | 2'30" | 2'30" | 2'35" | 2'35" | 2'35" |
| • temps de prise : (9) | 4'20" | 4'10" | 4'25" | 4'15" | 4'20" | 4'10" | 4'10" | 4'10" | 4'20" | 4'20" |
| Dureté Shore A | | | | | | | | | | |
| • 8 min : | 47 | 48 | 48 | 48 | 48 | 48 | 48 | 48 | 48 | 48 |
| • 24 heures : (10) | 49 | 49 | 49 | 49 | 49 | 49 | 49 | 49 | 49 | 49 |
| Angle de contact (11) | 29° | 25,7° | 31° | 33,5° | 32,5° | 25° | 29,3° | 29,5° | 29,6° | 28° |

Légende des tableaux 1, 2 et 3 :

(1) TA 1 : LINCOL PE 400 ML

(2) TA2 : RHODASURF ROX

(3) TA3 : TEGOPREN LP 111

(4) TA4 : ANTAROX FM 33

(5) TA5 : RHODASURF OT/2

(6) TA6 : TEGOPREN 5863 (polyorganosiloxane à fonctions polyéthers)

(7) % en poids dans A + B : c'est le % en poids dans la somme des composants A et B du bicomposant où le composant B est considéré sans prendre en compte le (ou les) agent(s) tensioactif(s).

(8) Rapport pondéral : il s'agit du rapport pondéral TA1/co-tensioactif (TA2, TA3, TA4 ou TA5) ; ou du rapport pondéral TA2/TA3.

(9) Les cinétiques de réticulation sont évaluées manuellement. On détermine les temps suivants :

- temps de travail : il correspond au temps pendant lequel le mélange des 2 composants A et B conserve un comportement fluide permettant l'application sur les modèles à reproduire ; au delà, le mélange acquière les caractéristiques d'un élastomère ;
- temps de prise : il correspond au temps, à 23°C, au bout duquel le durcissement est achevé, le mélange est sec au toucher et l'empreinte, par exemple dentaire, peut être retirée de la bouche.

(10) La dureté Shore A est mesurée d'une part 8 minutes après la réalisation du mélange des composants A et B (c'est-à-dire après 8 minutes de réticulation) et d'autre part 24 heures après la réalisation du mélange des 2 composants A et B (c'est-à-dire après 24 heures de réticulation), dans une atmosphère régulée à 23°C et à 50 % d'humidité relative, en opérant selon les indications de la norme DIN 53505 en utilisant un duromètre commercialisé sous la dénomination ZWICK 3140.H04 et des pions de 6 mm d'épaisseur.

(11) Angles de contact :

L'hydrophilie est mesurée, 10 minutes après la fin de la réticulation du matériau silicone ; ladite fin de réticulation est obtenue, en général, 15 minutes après la réalisation du mélange des 2 composants A et B. La méthode consiste à déposer une goutte d'eau d'environ 10 à 60 mm3, sur la surface d'un film, à base de l'élastomère silicone réticulé et à mesurer l'angle de contact θ à l'aide d'une caméra photographique (à agrandissement d'image) et d'un goniomètre consistant dans

l'appareil commercialisé sous la dénomination OLYMPUS DMS 300. Les angles sont mesurés 3 minutes après le dépôt de la goutte. Les valeurs indiquées sont des moyennes de 3 mesures. Les mesures sont réalisées dans une atmosphère régulée à 23°C et à 50 % d'humiditié relative. Les films d'élastomère silicone réticulé, de dimensions 7 cm x 3 cm x 0,2 cm, sont préparés par étalement d'un cordon de matériau silicone encore fluide à l'aide d'une raclette, et on laisse se poursuivre la réticulation jusqu'à son terme.

Les exemples 5, 8, 17, 18 et 20 rapportés supra montrent bien que la présente invention permet d'obtenir un matériau silicone ayant d'une part un très haut niveau d'hydrophilie marqué par l'obtention d'angles de contact aussi faibles que ceux allant de 25 à 27° et mieux de 25° à une valeur inférieure à 26°, et d'autre part d'excellentes propriétés mécaniques marquées notamment par l'obtention de valeurs de DSA à 8 minutes ou à 24 heures se situant dans la gamme 45-55.

**Revendications**

1. Matériau silicone qui comprend les constituants suivants :

   **I**. une composition POS réticulable par des réactions de polyaddition comprenant :

   (1) au moins un POS porteur de fonctions Si-alcényles aptes à réagir par des réactions d'addition avec les fonctions réticulantes Si-H d'un POS (2),
   (2) au moins un POS porteur de fonctions Si-H aptes à réagir avec les fonctions Si-alcényles du POS (1),
   (3) éventuellement au moins un POS non réactif, différent des POS(1) et (2), utilisable comme diluant,
   (4) un catalyseur des réactions de polyaddition,
   (5) une charge minérale renforçante particulaire ;

   **II.** un agent mouillant consistant dans un ou plusieurs agents(s) tensioactifs permettant de conférer un caractère hydrophile marqué à la surface du matériau silicone, avant comme après réticulation ;
   ledit matériau silicone étant **caractérisé en ce que** l'agent mouillant **II** est choisi dans le groupe constitué par :

   - (a) au moins un agent tensioactif non ionique consistant dans un ester obtenu par estérification d'un monoacide carboxylique saturé à chaîne linéaire ou ramifiée en $C_{10}$-$C_{14}$ par un poly(oxyalkylène)glycol contenant un nombre de motifs alkoxylés tel que la masse molaire Mw de l'ester se situe dans l'intervalle allant de 400 à 800 g/mole, le HLB dudit agent tensioactif ou du mélange d'agents tensioactifs allant de 6 à14;
   - une association synergique (a) + (b) où (b) désigne au moins un agent tensioactif non ionique consistant dans un alcool aliphatique saturé à chaîne linéaire ou ramifiée en $C_5$-$C_{16}$, polyalkoxylé contenant un nombre de motifs alkoxylés tel que la masse molaire Mw de l'alcool polyalkoxylé se situe dans l'intervalle allant de 200 à 1400 g/mole, le HLB dudit agent tensioactif (b) ou du mélange d'agents tensioactifs (b) allant de 6 à 16 ;
   - une association synergique (b1) + (b2) où : (b1) désigne au moins un agent tensioactif non ionique consistant dans un alcool aliphatique saturé à chaîne linéaire ou ramifiée en $C_{10}$-$C_{16}$, polyéthoxylé contenant de 6 à 10 motifs oxyéthylène (OE), le HLB dudit agent tensioactif (b1) ou du mélange d'agents tensioactifs (b1) allant de 12 à 16 ; (b2) désigne au moins un agent tensioactif non ionique consistant dans un alcool aliphatique saturé à chaîne linéaire ou ramifiée en $C_6$-$C_{13}$, polyalkoxylé contenant un nombre de motifs oxyéthylène (OE) et oxypropylène (OP) tel que la masse molaire Mw de l'alcool polyalkoxylé se situe dans l'intervalle allant de 500 à 1200 g/mole, le HLB dudit agent tensioactif (b2) ou du mélange d'agents tensioactifs (b2) allant de 6 à 16.

2. Matériau selon la revendication 1, **caractérisé en ce que** la quantité d'agent tensioactif ou celle d'un mélange d'agents tensioactifs, exprimée en % en poids par rapport à la masse totale de la composition POS **I** est égale ou

supérieure à 1 %, de préférence se situe dans l'intervalle allant de 1,2 à 4 %.

3. Matériau selon la revendication 1 ou 2, **caractérisé en ce que** l'on procède à l'utilisation, dans les quantités données supra, d'un agent mouillant choisi dans le groupe constitué par :

- (a1) au moins un agent tensioactif non ionique consistant dans un ester obtenu par estérification d'un monoacide carboxylique saturé à chaîne linéaire ou ramifiée en $C_{10}$-$C_{14}$ par un poly(oxyalkylène)glycol contenant de 7 à 11 motifs OE et/ou OP, le HLB dudit agent tensioactif ou du mélange d'agents tensioactifs allant de 10 à 14 ;
- une association synergique (a1) + (b1);
- une association synergique (a1) + (b2)
- une association synergique (a1) + (b3) où (b3) désigne au moins un agent tensioactif non ionique consistant dans un alcool aliphatique saturé à chaîne linéaire ou ramifiée en $C_{10}$-$C_{14}$, polyéthoxylé contenant de 1 à 4 motifs OE, le HLB dudit agent tensioactif (b3) ou du mélange d'agents tensioactifs (b3) allant de 6 à 10 ; et
- une association synergique (b1) + (b2).

4. Matériau selon la revendication 3, **caractérisé en ce que**, pour atteindre des angles de contact égaux ou inférieurs à 30°, on procède à l'utilisation d'un agent mouillant choisi dans le groupe formé par :

- une association synergique (a1) + (b1),
- une association synergique (a1) + (b2), et
- une association synergique (b1) + (b2),

où :

- la quantité du mélange d'agents tensioactifs se situe dans l'intervalle allant de 1,2 à 3%,
- le rapport pondéral (a1)/(b1) se situe dans l'intervalle allant de 0,5 à 1,5,
- le rapport pondéral (a1)/(b2) se situe dans l'intervalle allant de 0,5 à 7 avec les trois conditions selon lesquelles : (i) quand ledit rapport se situe dans l'intervalle allant de 0,5 à 1,2, alors la quantité du mélange d'agents tensioactifs est inférieure à 1,8 % ; (2i) quand ledit rapport se situe dans l'intervalle allant de 3 à 7, alors la quantité du mélange d'agents tensioactifs est égale ou supérieure à 1,8 % ; et (3i) quand ledit rapport se situe dans l'intervalle allant d'une valeur supérieure à 1,2 à une valeur inférieure à 3, alors la quantité du mélange d'agents tensioactifs est égale à une valeur quelconque prise dans l'intervalle donné supra allant de 1,2 à 3 %;
- le rapport pondéral (b1)/(b2) se situe dans l'intervalle allant de 0,5 à 1,5.

5. Matériau selon la revendication 4, **caractérisé en ce que**, pour atteindre des valeurs d'angle de contact aussi basses que celles allant de 25 à 27°, on procède à l'utilisation d'un agent mouillant consistant dans une association synergique (a1) + (b2),
où :

- la quantité du mélange d'agents tensioactifs se situe dans l'intervalle allant de 1,2 à 3%,
- le rapport pondéral (a1)/(b2) se situe dans l'intervalle allant de 0,7 à 1,7, avec les trois conditions selon lesquelles : (i) quand ledit rapport se situe dans l'intervalle allant de 0,7 à 1, alors la quantité du mélange d'agents tensioactifs est inférieure à 1,8 % ; (2i) quand ledit rapport se situe dans l'intervalle allant de 1,5 à 1,7, alors la quantité du mélange d'agents tensioactifs est égale ou supérieure à 1,8 % ; et (3i) quand ledit rapport se situe dans l'intervalle allant d'une valeur supérieure à 1 à une valeur inférieure à 1,5, alors la quantité du mélange d'agents tensioactifs est égale à une valeur quelconque prise dans l'intervalle donné supra allant de 1,2 à 3 %.

6. Matériau selon la revendication 4, **caractérisé en ce que**, pour atteindre des valeurs d'angles de contact aussi basses que celles allant de 25 à 27°, on procède à l'utilisation d'un agent mouillant consistant dans une association synergique (b1) + (b2), où :

- la quantité du mélange d'agents tensioactifs se situe dans l'intervalle allant de 1,2 à 3%,
- le rapport pondéral (b1)/(b2) se situe dans l'intervalle allant de 0,8 à 1,2.

7. Matériau selon l'une quelconque des revendications 1 à 6, **caractérisé en ce que** le POS (1) comprend :

(i) des motifs siloxyles de formule :

$$T_a\ Z_b\ SiO_{\frac{4-(a+b)}{2}} \qquad (1.1)$$

dans laquelle :

- T est un groupe alcényle en $C_2$-$C_6$,
- Z est un groupe hydrocarboné monovalent, exempt d'action défavorable sur l'activité du catalyseur et choisi, de préférence, parmi les groupes alkyles ayant de 1 à 8 atomes de carbone inclus, éventuellement substitués par au moins un atome d'halogène, ainsi que parmi les groupes aryles,
- a est 1 ou 2, b est 0, 1 ou 2 et a + b est compris entre 1 et 3,

et (2i) éventuellement des autres motifs siloxyles de formule :

$$Z_c\ SiO_{\frac{4-c}{2}} \qquad (1.2)$$

dans laquelle Z a la même signification que ci-dessus et c a une valeur comprise entre 0 et 3.

**8.** Matériau selon l'une quelconque des revendications 1 à 7, **caractérisé en ce que** le POS (2) comprend :

(i) des motifs siloxyles de formule :

$$H_d\ L_e\ SiO_{\frac{4-(d+e)}{2}} \qquad (2.1)$$

dans laquelle :

- L est un groupe hydrocarboné monovalent, exempt d'action défavorable sur l'activité du catalyseur et choisi parmi les groupes alkyles ayant de 1 à 8 atomes de carbone inclus, éventuellement substitués par au moins un atome d'halogène, ainsi que parmi les groupes aryles,
- d est 1 ou 2, e est 0, 1 ou 2, d + e a une valeur comprise entre 1 et 3,

et (2i) éventuellement des autres motifs siloxyles de formule moyenne :

$$L_g\ SiO_{\frac{4-g}{2}} \qquad (2.2)$$

dans laquelle L a la même signification que ci-dessus et g a une valeur comprise entre 0 et 3.

**9.** Matériau selon l'une quelconque des revendications 1 à 8, **caractérisé en ce qu'**il comprend en outre un ou plusieurs constituants complémentaires choisis dans le groupe comprenant :

(6) au moins un inhibiteur des réactions de polyaddition,
(7) une charge semi-renforçante ou de bourrage,
(8) un ou plusieurs agent(s) de coloration et/ou agent(s) édulcorant(s) et/ou arôme(s) et/ou composé(s) isotonique(s),
(9) un ou plusieurs biocide(s), et
(10) leurs mélanges.

**10.** Procédé de préparation d'un matériau silicone I + II selon l'une quelconque des revendications 1 à 9, **caractérisé en ce qu'**il consiste essentiellement à mélanger les ingrédients suivants :

(A) un ou plusieurs POS (1),
(B) un ou plusieurs POS (2),

(C) éventuellement un ou plusieurs POS (3),
(D) un catalyseur (4) des réactions de polyaddition,
(E) une charge minérale renforçante,
(F) éventuellement un ou plusieurs inhibiteur(s) (6),
(G) éventuellement une charge semi-renforçante ou de bourrage (7),
(H) éventuellement un ou plusieurs agent(s) (8),
(I) éventuellement un ou plusieurs agent(s) biocide(s) (9), et
(J) un ou plusieurs agent(s) tensioactif(s) II.

**11.** Procédé selon la revendication 9, **caractérisé par** les points suivants :

- on produit le matériau silicone sous forme d'un système à deux composants A et B destinés à être mis en contact l'un avec l'autre pour produire un élastomère réticulé par réactions de polyaddition entre les POS (1) et (2), et
- on fait en sorte que l'un seulement des composants A et B comprenne le catalyseur (D) et éventuellement l'un ou l'autre des POS (1) et (2).

**12.** Utilisation du matériau silicone **I** + **II** selon l'une quelconque des revendications 1 à 9 pour la prise d'empreintes.

**13.** Utilisation selon la revendication 12, **caractérisée en ce qu'**elle consiste à faire en sorte que la réticulation de l'élastomère silicone s'initie par mélange des composants A et B, à prendre l'empreinte et à laisser se poursuivre la réticulation jusqu'à ce que l'élastomère ait suffisamment réticulé et soit suffisamment dur.

**14.** Utilisation du matériau silicone I + II selon l'une quelconque des revendications 1 à 9 pour pour la fabrication de pièces moulées, autres que des masses de duplication dans les applications dentaires, capables de développer en surface un caractère hydrophile et/ou antistatique marqué.

**15.** Utilisation selon la revendication 14, **caractérisée en ce que** les pièces moulées, autres que des masses de duplication dans les applications dentaires, sont des tampons tels que ceux utilisés dans les techniques de tampographie et des rouleaux de photocopieurs

**16.** Utilisation selon la revendication 15, **caractérisée en ce qu'**elle consiste à fabriquer des tampons tels que ceux utilisés dans les techniques de tampographie, en faisant en sorte que la réticulation de l'élastomère silicone s'initie par mélange des composants A et B, à conformer par moulage un objet ayant la forme du tampon souhaité et à laisser se poursuivre la réticulation jusqu'à ce que l'élastomère ait suffisamment réticulé et soit suffisamment dur.

**17.** Matériau silicone réticulable en élastomère silicone par des réactions de polyaddition, ledit matériau étant susceptible d'être obtenu par le procédé selon la revendication 10 ou 11, **caractérisé par** les propriétés suivantes prises en combinaison, lesdites propriétés étant celles mesurées après réticulation dans une atmosphère régulée à 23°C et à 50 % d'humidité relative :

- un caractère hydrophile marqué par une valeur d'angle de goutte $\theta$ égale ou inférieure à 27°,
- et une DSA à 8 minutes ou à 24 heures se situant dans la gamme 45-55.

**18.** Utilisation du matériau selon la revendication 17 pour la prise d'empreintes.

**19.** Utilisation du matériau selon la revendication 17 pour la fabrication de pièces moulées, autres que des masses de duplication dans les applications dentaires, capables de développer en surface un caractère hydrophile et/ou antistatique marqué.

**20.** Utilisation selon la revendication 19, **caractérisée en ce que** les pièces moulées, autres que des masses de duplication dans les applications dentaires, sont des tampons tels que ceux utilisés dans les techniques de tampographie et des rouleaux de photocopieurs

**Patentansprüche**

**1.** Siliconmaterial, das die folgenden Bestandteile umfaßt:

I. eine Zusammensetzung POS, vernetzbar durch Reaktionen der Polyaddition, umfassend:

(1) mindestens ein POS als Träger von Funktionen Si-Alkenyl, die fähig sind, durch Additionsreaktionen mit den vernetzenden Funktionen Si-H eines POS (2) zu reagieren,
(2) mindestens ein POS als Träger von Funktionen Si-H, die fähig sind, mit den Funktionen Si-Alkenyl des POS (1) zu reagieren,
(3) gegebenenfalls mindestens ein nicht reaktives POS, das sich von den POS (1) und (2) unterscheidet, verwendbar als Verdünnungsmittel,
(4) einen Katalysator für die Reaktionen der Polyaddition,
(5) einen teilchenförmigen verstärkenden mineralischen Füllstoff;

II. ein Netzmittel, bestehend aus einem oder mehreren oberflächenaktiven Mittel(n), das ermöglicht, der Oberfläche des Siliconmaterials einen deutlichen hydrophilen Charakter vor wie nach der Vernetzung zu verleihen,

wobei das genannte Siliconmaterial **dadurch gekennzeichnet ist, daß** das Netzmittel II aus der Gruppe gewählt wird, die gebildet wird durch:

- (a) mindestens ein nichtionisches oberflächenaktives Mittel, bestehend aus einem Ester, erhalten durch Veresterung einer gesättigten Monocarbonsäure mit linearer oder verzweigter Kette und 10 bis 14 Kohlenstoffatomen durch ein Poly(oxyalkylen)-glycol, das eine solche Anzahl von Struktureinheiten Alkoxyl enthält, daß sich die Molmasse Mw des Esters in dem Intervall befindet, das von 400 bis 800 g/mol reicht, die HLB des genannten oberflächenaktiven Mittels oder der Mischung von oberflächenaktiven Mitteln von 6 bis 14 reicht;
- eine synergistische Assoziation (a) + (b), worin (b) mindestens ein nichtionisches oberflächenaktives Mittel bezeichnet, bestehend aus einem polyalkoxylierten gesättigten aliphatischen Alkohol mit linearer oder verzweigter Kette und 5 bis 16 Kohlenstoffatomen, der eine solche Anzahl von Struktureinheiten Alkoxyl enthält, daß sich die Molmasse Mw des polyalkoxylierten Alkohols in dem Intervall befindet, das von 200 bis 1400 g/mol reicht, die HLB des genannten oberflächenaktiven Mittels (b) oder der Mischung von oberflächenaktiven Mitteln (b) von 6 bis 16 reicht;
- eine synergistische Assoziation (b1) + (b2), worin (b1) mindestens ein nichtionisches oberflächenaktives Mittel bezeichnet, bestehend aus einem polyethoxylierten gesättigten aliphatischen Alkohol mit linearer oder verzweigter Kette und 10 bis 18 Kohlenstoffatomen, der 6 bis 10 Struktureinheiten Oxyethylen (OE) enthält, und die HLB des genannten oberflächenaktiven Mittels (b1) oder der Mischung von oberflächenaktiven Mitteln (b1) von 12 bis 16 reicht; und (b2) mindestens ein nichtionisches oberflächenaktives Mittel bezeichnet, bestehend aus einem polyalkoxylierten gesättigten aliphatischen Alkohol mit linearer oder verzweigter Kette und 6 bis 13 Kohlenstoffatomen, der eine solche Anzahl von Struktureinheiten Oxyethylen (OE) und Oxypropylen (OP) enthält, daß sich die Molmasse Mw des polyalkoxylierten Alkohols in dem Intervall befindet, das von 500 bis 1200 g/mol reicht, die HLB des genannten oberflächenaktiven Mittels (b2) oder der Mischung von oberflächenaktiven Mitteln (b2) von 6 bis 16 reicht.

2. Material nach Anspruch 1, **dadurch gekennzeichnet, daß** die Menge des oberflächenaktiven Mittels oder der Mischung von oberflächenaktiven Mitteln, ausgedrückt in Gew.-%, bezogen auf die Gesamtmasse der Zusammensetzung POS I, gleich oder höher als 1 % beträgt und sich vorzugsweise im Intervall befindet, das von 1,2 % bis 4 % reicht.

3. Material nach Anspruch 1 oder 2, **dadurch gekennzeichnet, daß** man bei der Verwendung eines Netzmittels in den oben angegebenen Mengen so vorgeht, daß es gewählt wird aus der Gruppe, die gebildet wird durch:

- (a1) mindestens ein nichtionisches oberflächenaktives Mittel, bestehend aus einem Ester, erhalten durch Veresterung einer gesättigten Monocarbonsäure mit linearer oder verzweigter Kette und 10 bis 14 Kohlenstoffatomen durch ein Poly(oxyalkylen)-glycol, das 7 bis 11 Struktureinheiten OE und/oder OP enthält, und die HLB des genannten oberflächenaktiven Mittels oder der Mischung von oberflächenaktiven Mitteln von 10 bis 14 reicht;
- eine synergistische Assoziation (a1) + (b1),
- eine synergistische Assoziation (a1) + (b2),
- eine synergistische Assoziation (a1) + (b3), worin (b3) mindestens ein nichtionisches oberflächenaktives Mittel bezeichnet, bestehend aus einem polyethoxylierten gesättigten aliphatischen Alkohol mit linearer oder verzweigter Kette und 10 bis 14 Kohlenstoffatomen, der 1 bis 4 Struktureinheiten OE enthält, und die HLB des genannten oberflächenaktiven Mittels (b3) oder der Mischung von oberflächenaktiven Mitteln (b3) von 6 bis

10 reicht, und
- eine synergistische Assoziation (b1) + (b2).

**4.** Material nach Anspruch 3, **dadurch gekennzeichnet, daß** man zum Erreichen von Kontaktwinkeln von gleich oder unter 30° bei der Verwendung eines Netzmittels so vorgeht, daß es gewählt wird aus der Gruppe, die gebildet wird durch:

- eine synergistische Assoziation (a1) + (b1),
- eine synergistische Assoziation (a1) + (b2), und
- eine synergistische Assoziation (b1) + (b2),

worin:

- · sich die Menge der Mischung von oberflächenaktiven Mitteln in dem Intervall befindet, das von 1,2 % bis 3 % reicht,
- · sich das Gewichtsverhältnis (a1)/(b1) in dem Intervall befindet, das von 0,5 bis 1,5 reicht,
- · sich das Gewichtsverhältnis (a1)/(b2) in dem Intervall befindet, das von 0,5 bis 7 reicht, mit den drei Bedingungen, gemäß denen: (i) wenn das genannte Verhältnis in dem Intervall liegt, das von 0,5 bis 1,2 reicht, dann die Menge der Mischung von oberflächenaktiven Mitteln unter 1,8 % beträgt; (2i) wenn das genannte Verhältnis in dem Intervall liegt, das von 3 bis 7 reicht, dann die Menge der Mischung von oberflächenaktiven Mitteln gleich oder über 1,8 % beträgt; und (3i) wenn das genannte Verhältnis in dem Intervall liegt, das von einem Wert von über 1,2 bis zu einem Wert von unter 3 reicht, dann die Menge der Mischung von oberflächenaktiven Mitteln gleich irgendeinem Wert ist, der aus dem oben genannten Intervall genommen wird, das von 1,2 % bis 3 % reicht;
- · sich das Gewichtsverhältnis (b1)/(b2) in dem Intervall befindet, das von 0,5 bis 1,5 reicht.

**5.** Material nach Anspruch 4, **dadurch gekennzeichnet, daß** man zum Erreichen von ebenso niedrigen Werten des Kontaktwinkels wie denen, die von 25° bis 27° reichen, bei der Verwendung eines Netzmittels so vorgeht, daß es aus einer synergistischen Assoziation (a1) + (b2) gebildet wird,
worin:

- · sich die Menge der Mischung von oberflächenaktiven Mitteln in dem Intervall befindet, das von 1,2 % bis 3 % reicht,
- · sich das Gewichtsverhältnis (a1)/(b2) in dem Intervall befindet, das von 0,7 bis 1,7 reicht, mit den drei Bedingungen, gemäß denen: (i) wenn das genannte Verhältnis in dem Intervall liegt, das von 0,7 bis 1 reicht, dann die Menge der Mischung von oberflächenaktiven Mitteln unter 1,8 % beträgt; (2i) wenn das genannte Verhältnis in dem Intervall liegt, das von 1,5 bis 1,7 reicht, dann die Menge der Mischung von oberflächenaktiven Mitteln gleich oder über 1,8 % beträgt; und (3i) wenn das genannte Verhältnis in dem Intervall liegt, das von einem Wert von über 1 bis zu einem Wert von unter 1,5 reicht, dann die Menge der Mischung von oberflächenaktiven Mitteln gleich irgendeinem Wert ist, der aus dem oben genannten Intervall genommen wird, das von 1,2 % bis 3 % reicht.

**6.** Material nach Anspruch 4, **dadurch gekennzeichnet, daß** man zum Erreichen von ebenso niedrigen Werten des Kontaktwinkels wie denen, die von 25° bis 27° reichen, bei der Verwendung eines Netzmittels so vorgeht, daß es aus einer synergistischen Assoziation (b1) + (b2) gebildet wird,
worin:

- · sich die Menge der Mischung von oberflächenaktiven Mitteln in dem Intervall befindet, das von 1,2 % bis 3 % reicht,
- · sich das Gewichtsverhältnis (b1)/(b2) in dem Intervall befindet, das von 0,8 bis 1,2 reicht.

**7.** Material nach irgendeinem der Ansprüche 1 bis 6, **dadurch gekennzeichnet, daß** das POS (1) umfaßt:

(i) Struktureinheiten Siloxyl der Formel (1.1)

$$T_aZ_bSiO_{\frac{4-(a+b)}{2}} \qquad (1.1)$$

in der

- T eine Gruppe Alkenyl mit 2 bis 6 Kohlenstoffatomen ist,
- Z eine monovalente Kohlenwasserstoffgruppe darstellt, die frei ist von einer ungünstigen Wirkung auf die Aktivität des Katalysators und vorzugsweise ausgewählt wird unter den Gruppen Alkyl mit einschließlich 1 bis 8 Kohlenstoffatomen, gegebenenfalls substituiert durch mindestens ein Halogenatom, sowie unter den Arylgruppen,
- a 1 oder 2 ist, b 0, 1 oder 2 ist und a + b zwischen einschließlich 1 und 3 beträgt,

und (2i) gegebenenfalls andere Struktureinheiten Siloxyl der Formel (1.2)

$$Z_cSiO_{\frac{4-c}{2}} \quad (1.2)$$

in der
Z die gleiche Bedeutung wie vorstehend aufweist und c einen Wert zwischen einschließlich 0 und 3 besitzt.

**8.** Material nach irgendeinem der Ansprüche 1 bis 7, **dadurch gekennzeichnet, daß** das POS (2) umfaßt:

(i) Struktureinheiten Siloxyl der Formel (2.1)

$$H_dL_eSiO_{\frac{4-(d+e)}{2}} \quad (2.1)$$

in der

- L eine monovalente Kohlenwasserstoffgruppe darstellt, die frei ist von einer ungünstigen Wirkung auf die Aktivität des Katalysators und ausgewählt wird unter den Gruppen Alkyl mit einschließlich 1 bis 8 Kohlenstoffatomen, gegebenenfalls substituiert durch mindestens ein Halogenatom, sowie unter den Arylgruppen,
- d 1 oder 2 ist, e 0, 1 oder 2 ist und d + e einen Wert zwischen einschließlich 1 und 3 besitzt,

und (2i) gegebenenfalls andere Struktureinheiten Siloxyl der mittleren Formel (2.2)

$$L_gSiO_{\frac{4-g}{2}} \quad (2.2)$$

in der
L die gleiche Bedeutung wie vorstehend aufweist und g einen Wert zwischen einschließlich 0 und 3 besitzt.

**9.** Material nach irgendeinem der Ansprüche 1 bis 8, **dadurch gekennzeichnet, daß** es außerdem einen oder mehrere zusätzliche Bestandteile enthält, gewählt aus der Gruppe, die umfaßt:

(6) mindestens einen Inhibitor für die Reaktionen der Polyaddition,
(7) einen halbverstärkenden Füllstoff oder eine Füllung,
(8) ein oder mehrere Mittel zur Färbung und/oder Süßungsmittel und/oder Aromastoff(e) und/oder isotonische Verbindung(en),
(9) ein oder mehrere Biozid(e), und
(10) ihre Mischungen.

**10.** Verfahren zur Herstellung eines Siliconmaterials I + II nach irgendeinem der Ansprüche 1 bis 9, **dadurch gekennzeichnet, daß** es im wesentlichen darin besteht, die folgenden Bestandteile zu vermischen:

(A) ein oder mehrere POS (1),
(B) ein oder mehrere POS (2),
(C) gegebenenfalls ein oder mehrere POS (3),

(D) einen Katalysator (4) für die Reaktionen der Polyaddition,
(E) einen verstärkenden mineralischen Füllstoff,
(F) gegebenenfalls einen oder mehrere Inhibitor(en) (6),
(G) gegebenenfalls einen halbverstärkenden Füllstoff oder eine Füllung (7),
(H) gegebenenfalls ein oder mehrere Mittel (8),
(I) gegebenenfalls ein oder mehrere biozide(s) Mittel (9), und
(J) ein oder mehrere oberflächenaktive(s) Mittel II.

**11.** Verfahren nach Anspruch 9, **gekennzeichnet durch** die folgenden Punkte:

- man stellt das Siliconmaterial in Form eines Systems mit zwei Komponenten A und B her, die dazu vorgesehen sind, miteinander in Kontakt zu treten, um ein Elastomer zu erzeugen, das **durch** Reaktionen der Polyaddition zwischen den POS (1) und (2) vernetzt, und
- daß man in der Weise vorgeht, daß nur ein einziger der Bestandteile A und B den Katalysator (D) und gegebenenfalls das eine oder andere POS (1) und (2) umfaßt.

**12.** Verwendung des Siliconmaterials I + II nach irgendeinem der Ansprüche 1 bis 9 für das Abformen.

**13.** Verwendung nach Anspruch 12, **dadurch gekennzeichnet, daß** sie darin besteht, in einer solchen Weise vorzugehen, daß die Vernetzung des Siliconelastomers durch Vermischen der Bestandteile A und B in Gang gebracht, die Abformung vorgenommen wird und man dann die Vernetzung weiter fortschreiten läßt, bis das Elastomer ausreichend vernetzt wurde und ausreichend hart geworden ist.

**14.** Verwendung des Siliconmaterials I + II nach irgendeinem der Ansprüche 1 bis 9 für die Herstellung von Gießteilen, die anders sind als Massen zur Vervielfältigung bei dentalen Anwendungen und die fähig sind, an der Oberfläche einen deutlichen hydrophilen und/oder antistatischen Charakter zu entwickeln.

**15.** Verwendung nach Anspruch 14, **dadurch gekennzeichnet, daß** die Gießteile, die anders sind als Massen zur Vervielfältigung bei dentalen Anwendungen, Pfropfen bzw. Stempel sind, wie sie bei den Techniken der Tampographie und bei Walzen von Photokopierern verwendet werden.

**16.** Verwendung nach Anspruch 15, **dadurch gekennzeichnet, daß** sie darin besteht, Stempel (Tampons) herzustellen, wie sie bei den Techniken der Tampographie verwendet werden, indem man in einer solchen Weise vorgeht, daß die Vernetzung des Siliconelastomers durch Vermischen der Bestandteile A und B durch Formgießen eines Objektes, das die Form des gewünschten Stempels (Tampon) besitzt, in Gang gebracht wird und man dann die Vernetzung weiter fortschreiten läßt, bis das Elastomer ausreichend vernetzt wurde und ausreichend hart geworden ist.

**17.** Siliconmaterial, vernetzbar zu einem Siliconelastomer durch Reaktionen der Polyaddition, wobei das genannte Material geeignet ist, gemäß dem Verfahren nach Anspruch 10 oder 11 erhalten zu werden, **gekennzeichnet durch** die folgenden, in Kombination betrachteten Eigenschaften, wobei diese Eigenschaften solche sind, wie sie nach der Vernetzung in einer geregelten Atmosphäre bei 23 °C und 50 % relativer Feuchte gemessen werden:

- einen hydrophilen Charakter, dargestellt **durch** einen Wert des Tropfenwinkels $\Theta$ von gleich oder unter 27°, und
- eine DSA bei 8 Minuten oder 24 Stunden, die sich im Bereich von 45-55 befindet.

**18.** Verwendung des Materials nach Anspruch 17 für Abformungen.

**19.** Verwendung des Materials nach Anspruch 17 für die Herstellung von Gießteilen, die anders sind als Massen zur Vervielfältigung bei dentalen Anwendungen und die fähig sind, an der Oberfläche einen deutlichen hydrophilen und/oder antistatischen Charakter zu entwickeln.

**20.** Verwendung nach Anspruch 19, **dadurch gekennzeichnet, daß** die Gießteile, die anders sind als Massen zur Vervielfältigung bei dentalen Anwendungen, Stempel (Tampons) sind, wie sie bei den Techniken der Tampographie und bei Walzen von Photokopierern verwendet werden.

## Claims

1. Silicone material which comprises the following constituents:

I. a POS composition crosslinkable by polyaddition reactions comprising:

(1) at least one POS composition carrying Si-alkenyl functional groups which are capable of reacting by addition reactions with the Si-H crosslinking functional groups of a POS(2) composition,
(2) at least one POS composition carrying Si-H functional groups which are capable of reacting with the Si-alkenyl functional groups of the POS(1) composition,
(3) optionally, at least one unreactive POS composition, differing from the POS(1) and POS(2) compositions, which can be used as a diluent,
(4) a catalyst for catalysing the polyaddition reactions,
(5) a particulate reinforcing mineral filler;

II. a wetting agent consisting of one or more surfactants allowing the surface of the silicone material to be given a pronounced hydrophilic character, both before and after crosslinking;
the said silicone material being **characterized in that** the wetting agent II is chosen from the group consisting of:

- (a) at least one nonionic surfactant consisting of an ester obtained by esterification of a saturated monocarboxylic acid having a $C_{10}$-$C_{14}$ linear or branched chain by a poly(oxyalkylene) glycol containing a number of alkoxylated units such that the molar mass $M_w$ of the ester lies within the 400 to 800 g/mol range, the HLB of the said surfactant or of the mixture of surfactants ranging from 6 to 14;
- a synergistic combination (a) + (b), where (b) denotes at least one nonionic surfactant consisting of a polyalkoxylated saturated aliphatic alcohol having a $C_5$-$C_{16}$ linear or branched chain, containing a number of alkoxylated units such that the molar mass $M_w$ of the polyalkoxylated alcohol lies within the 200 to 1400 g/mol range, the HLB of the said surfactant (b) or of the mixture of surfactants (b) ranging from 6 to 16; and
- a synergistic combination (b1) + (b2), where (b1) denotes at least one nonionic surfactant consisting of a polyethoxylated saturated aliphatic alcohol having a $C_{10}$-$C_{16}$ linear or branched chain, containing from 6 to 10 ethylene oxide (EO) units, the HLB of the said surfactant (b1) or of the mixture of surfactants (b1) ranging from 12 to 16, and (b2) denotes at least one nonionic surfactant consisting of a polyalkoxylated saturated aliphatic alcohol having a $C_6$-$C_{13}$ linear or branched chain, containing a number of ethylene oxide (EO) and propylene oxide (PO) units such that the molar mass $M_w$ of the polyalkoxylated alcohol lies within the 500 to 1200 g/mol range, the HLB of the said surfactant (b2) or of the mixture of surfactants (b2) ranging from 6 to 16.

2. Material according to claim 1, **characterized in that** the amount of surfactant or that of a mixture of surfactants, expressed in % by weight with respect to the total mass of the POS I composition, is equal to or greater than 1% and preferably lies within the 1.2 to 4% range.

3. Material according to claim 1 or 2, **characterized by** the use, in the amounts given above, of a wetting agent chosen from the group consisting of:

- (a1) at least one nonionic surfactant consisting of an ester obtained by esterification of a saturated monocarboxylic acid having a $C_{10}$-$C_{14}$ linear or branched chain by a poly(oxyalkylene) glycol containing from 7 to 11 EO and/or PO units, the HLB of the said surfactant or of the mixture of surfactants ranging from 10 to 14;
- a synergistic combination (a1) + (b1);
- a synergistic combination (a1) + (b2);
- a synergistic combination (a1) + (b3), where (b3) denotes at least one nonionic surfactant consisting of a polyethoxylated saturated aliphatic alcohol having a $C_{10}$-$C_{14}$ linear or branched chain, containing from 1 to 4 EO units, the HLB of the said surfactant (b3) or of the mixture of surfactants (b3) ranging from 6 to 10; and
- a synergistic combination (b1) + (b2).

4. Material according to claim 3, **characterized, in** order to achieve contact angles equal to or less than 30°, by the use of a wetting agent chosen from the group formed by:

- a synergistic combination (a1) + (b1);

- a synergistic combination (a1) + (b2); and
- a synergistic combination (b1) + (b2);

where:

- the amount of the surfactant mixture lies within the 1.2 to 3% range;
- the (a1)/(b1) weight ratio lies within the 0.5 to 1.5 range;
- the (a1)/(b2) weight ratio lies within the 0.5 to 7 range with the three conditions whereby: (i) when the said ratio lies within the 0.5 to 1.2 range, then the amount of the surfactant mixture is less than 1.8%; (2i) when the said ratio lies within the 3 to 7 range, then the amount of the surfactant mixture is equal to or greater than 1.8%; and (3i) when the said ratio lies within the range going from a value greater than 1.2 to a value less than 3, then the amount of the surfactant mixture is equal to any value taken within the 1.2 to 3% range given above;
- the (b1)/(b2) weight ratio lies within the 0.5 to 1.5 range.

**5.** Material according to claim 4, **characterized, in** order to achieve contact angle values as low as those ranging from 25 to 27°, by the use of a wetting agent consisting of a synergistic combination (a1) + (b2), where:

- the amount of the surfactant mixture lies within the 1.2 to 3% range;
- the (a1) / (b2) weight ratio lies within the 0.7 to 1.7 range, with the three conditions whereby: (i) when the said ratio lies within the 0.7 to 1 range, then the amount of the surfactant mixture is less than 1.8%; (2i) when the said ratio lies within the 1.5 to 1.7 range, then the amount of the surfactant mixture is equal to or greater than 1.8%; and (3i) when the said ratio lies within the range going from a value greater than 1 to a value less than 1.5, then the amount of the surfactant mixture is equal to any value taken within the 1.2 to 3% range given above.

**6.** Material according to claim 4, **characterized, in** order to achieve contact angle values as low as those ranging from 25 to 27°, by the use of a wetting agent consisting of a synergistic combination (b1) + (b2), where:

- the amount of the surfactant mixture lies within the 1.2 to 3% range;
- the (b1)/(b2) weight ratio lies within the 0.8 to 1.2 range.

**7.** Material according to any one of claims 1 to 6, **characterized in that** the POS(1) composition comprises:

(i) siloxyl units of formula:

$$T_a Z_b SiO_{\frac{4-(a+b)}{2}} \qquad (1.1)$$

in which:

- T is a $C_2$-$C_6$ alkenyl group;
- Z is a monovalent hydrocarbon group, not having any action unfavourable to the activity of the catalyst and preferably chosen from alkyl groups having from 1 to 8 carbon atoms inclusive, optionally substituted with at least one halogen atom, and from aryl groups;
- a is 1 or 2, b is 0, 1 or 2 and a + b is between 1 and 3; and

(2i) optionally other siloxyl units of formula:

$$Z_c SiO_{\frac{4-c}{2}} \qquad (1.2)$$

in which Z has the same meaning as above and c has a value of between 0 and 3.

**8.** Material according to any one of claims 1 to 7, **characterized in that** the POS(2) composition comprises:

(i) siloxyl units of formula:

$$H_dL_eSiO_{\frac{4-(d+e)}{2}} \quad (2.1)$$

in which:

- L is a monovalent hydrocarbon group, having no action unfavourable to the activity of the catalyst and chosen from alkyl groups having from 1 to 8 carbon atoms inclusive, optionally substituted with at least one halogen atom, and from aryl groups;
- d is 1 or 2, e is 0, 1 or 2 and d + e has a value of between 1 and 3; and

(2i) optionally other siloxyl units of average formula:

$$L_gSiO_{\frac{4-g}{2}} \quad (2.2)$$

in which L has the same meaning as above and g has a value of between 0 and 3.

**9.** Material according to any one of claims 1 to 8, **characterized in that** it furthermore includes one or more complementary constituents chosen from the group comprising:

(6) at least one inhibitor for the polyaddition reactions;
(7) a semi-reinforcing or bulking filler;
(8) one or more colouring agents and/or sweeteners and/or flavours and/or isotonic compounds;
(9) one or more biocides; and
(10) mixtures thereof.

**10.** Process for preparing a silicone material I + II according to any one of claims 1 to 9, **characterized in that** it essentially consists in mixing the following ingredients:

(A) one or more POS(1) compositions;
(B) one or more POS(2) compositions;
(C) optionally, one or more POS(3) compositions;
(D) a catalyst (4) for catalysing the polyaddition reactions;
(E) a reinforcing mineral filler;
(F) optionally, one or more inhibitors (6);
(G) optionally, a semi-reinforcing or bulking filler (7);
(H) optionally, one or more agents (8);
(I) optionally, one or more biocidal agents (9); and
(J) one or more surfactants II.

**11.** Process according to claim 9, **characterized by** the following points:

- the silicone material is produced in the form of a system based on two components A and B intended to be brought into contact with each other in order to produce an elastomer crosslinked by polyaddition reactions between the POS(1) and (2) compositions; and
- measures are taken to ensure that only one of the components A and B contains the catalyst (D) and possibly one or other of the POS(1) and (2) compositions.

**12.** Use of the silicone material I + II according to any one of claims 1 to 9 for taking impressions.

**13.** Use according to claim 12, **characterized in that** it consists in taking measures to ensure that the crosslinking of the silicone elastomer is initiated by mixing the components A and B together, in taking the impression and in allowing the crosslinking to continue until the elastomer is sufficiently crosslinked and sufficiently hard.

**14.** Use of the silicone material I + II according to any one of claims 1 to 9 for the manufacture of moulded parts, other

than duplicates in dental applications, which are capable of developing a pronounced hydrophilic and/or antistatic character on the surface.

**15.** Use according to claim 14, **characterized in that** the moulded parts, other than duplicates in dental applications, are pads, such as those used in pad printing techniques, and rollers of photocopiers.

**16.** Use according to claim 15, **characterized in that** it consists in manufacturing pads such as those employed in pad printing techniques, by taking measures to ensure that the crosslinking of the silicone elastomer is initiated by mixing the components A and B together, in forming, by moulding, an object having the shape of the desired pad and in allowing the crosslinking to continue until the elastomer is sufficiently crosslinked and sufficiently hard.

**17.** Crosslinkable silicone material made of a silicone elastomer by polyaddition reactions, the said material being able to be obtained by the process according to claim 10 or 11, **characterized by** the following properties taken in combination, the said properties being those measured after crosslinking in an atmosphere controlled to 23°C and 50% relative humidity:

- a pronounced hydrophilic character indicated by a value of the drop angle θ equal to or less than 27°; and
- a Shore A hardness at 8 minutes or at 24 hours lying within the 45-55 range.

**18.** Use of the material according to claim 17 for taking impressions.

**19.** Use of the material according to claim 17 for the manufacture of moulded parts, other than duplicates in dental applications, which are capable of developing a pronounced hydrophilic and/or antistatic character on the surface.

**20.** Use according to claim 19, **characterized in that** the moulded parts, other than duplicates in dental applications, are pads, such as those used in pad printing techniques, and rollers of photocopiers.

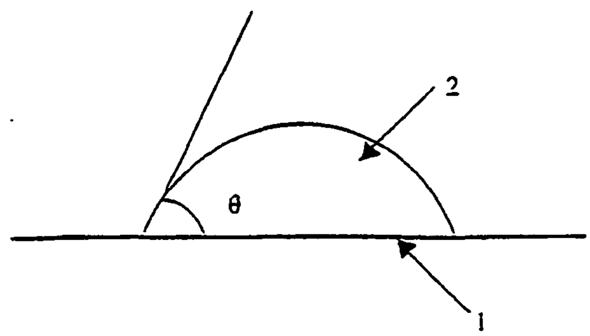


Fig 1